(19)

Europäisches
Patentamt
European
Patent Office
Office européen
des brevets

(11) **EP 2 340 814 A2**

(12) **EUROPEAN PATENT APPLICATION**

(43) Date of publication:
06.07.2011 Bulletin 2011/27

(51) Int Cl.:
*A61K 9/20* (2006.01)   *A61K 9/28* (2006.01)
*A61K 31/60* (2006.01)

(21) Application number: **10197404.6**

(22) Date of filing: **30.12.2010**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **31.12.2009   IN DE27602009**

(71) Applicant: **Ranbaxy Laboratories Limited
Gurgaon - 122001, Haryana (IN)**

(72) Inventors:
• **Jha, Romesh**
**New Delhi**
**110049 Delhi (IN)**
• **Agarwal, Ravindra**
**313002 Rajasthan (IN)**
• **Raghuvanshi, Rajeev Singh**
**122001 Gurgaon,**
**Haryana (IN)**

(74) Representative: **Cronin, Brian Harold John et al
Cronin Intellectual Property
Chemin de Précossy 31
CH-1260 Nyon (CH)**

(54)    **A stable aspirin tablet and process of preparation thereof**

(57)    A stable aspirin tablet is disclosed. The tablet is prepared under the controlled conditions of humidity. The free salicylic acid generated in the tablet is not more than 3.0% when the tablet is kept for stability testing for 6 months.

EP 2 340 814 A2

**Description**

Technical Field of the Invention

The present invention relates to a stable aspirin tablet prepared under the controlled conditions of humidity.

Background of the Invention

Aspirin is an analgesic, antipyretic and anti-inflammatory drug, it also reduces platelet aggregation. Aspirin is a cyclooxygenase (prostaglandin synthetase) inhibitor thereby reducing the synthesis of prostaglandins and thromboxanes. These effects are thought to be how most cells can synthesize new cyclooxygenase, but platelets cannot. Therefore, aspirin causes an irreversible effect on platelet aggregation. Aspirin has been shown to decrease the clinical symptoms of experimentally induced anaphylaxis in calves and ponies.

Chemically aspirin is acetylsalicylic acid or ASA, the salicylate ester of acetic acid. The compound occurs as a white, crystalline powder or tabular or needle-like crystals. It is a weak acid with a pKa of 3.5. Aspirin is slightly soluble in water and is freely soluble in alcohol. Each gram of aspirin contains approximately 760 mg of salicylate.

Two major concerns associated with aspirin tablets are;

a) Side effects associated with administration of aspirin and;

b) Stability of aspirin during storage.

Aspirin which is salicylic acid ester becomes therapeutically active upon hydrolysis to form free salicylic acid (FSA). FSA is very irritating to gastric mucosa and is associated with dyspepsia and gastric bleeding. Enteric coating of tablets and especially aspirin tablets is undertaken in the art to prevent the gastric irritation in individuals which often follow the administration of such tablets which are uncoated.

The chemical stability of a drug is of great importance since it becomes less effective as it undergoes degradation. Also, drug decomposition may yield toxic byproducts that are harmful to the patient. Aspirin undergoes hydrolysis with the resultant degradation products being salicylic acid and acetic acid. The acetic acid seems to impart a vinegar taste to the coated tablets after standing.

Aspirin → Salicylic Acid + Acetic Acid

The rate of this reaction is said to be second order, as it is dependent not only upon the aspirin concentration, but upon solution pH. At pH = 7.5 the rate expression for the hydrolysis of aspirin may be written:

$$\frac{-d[A]}{dt} = K[A][OH^-] \qquad (1)$$

where

[A] = the concentration of aspirin (acetyl salicylic acid) (mol/L)
[OH⁻] = hydroxyl ion concentration (mol/L)
K = second order rate constant (L/min·mol)
t = time (min)

Several attempts have been made in the art to improve the stability of aspirin tablets. Stability of aspirin tablet was improved by combining certain agents with aspirin which prevent degradation of aspirin.

Early attempts to utilize silica or other water absorbents as water scavengers or to use solubilize agents or disintegrating agents prior to applying a coating film on the tablet surface proved fruitless since, on long storage, atmospheric moisture caused hydrolysis and formation of FSA. Other water scavengers, such as talcum powder, sodium lauryl sulfate, glycine, magnesium carbonate and the like, were similarly disappointing. U.S. Patent Nos. 4,975,283 and 4,900,559 disclose the addition of glutamic acid to the aspirin. U.S. Patent No. 4,716,042 discloses the incorporation of a small amount of citric acid, alginic acid, glutamic acid or mixtures of any thereof.

Further, some digestive enzymes were tried to inhibit the degradation of aspirin. EP Patent No. 0 768 082 discloses the use of pepsin as an agent to improve the stability of aspirin.

Improving stability of aspirin tablets was also tried without using any chemical agent to be incorporated with drug. In this effort coating of aspirin tablet with a thin layer of methyl cellulose or equivalent coating was tried. These too were found of little value to avoid stabilizing aspirin products against hydrolysis.

It is known in the art that processing parameters and conditions during the preparation affect the stability of final drug formulation. The stability of final formulation can be increased by optimizing these parameters. An article published by Ruotsalainen M, et al., Pharm Dev Technol. (2003);8(4):443-51, discloses the influence of an aqueous film coating process on the morphology and storage stability of hydroxypropyl methylcellulose-coated tablets containing a moisture-labile model drug (acetylsalicylic acid, ASA). Coating parameters studied were inlet air absolute humidity 5 $g/m^3$ and 12 $g/m^3$, spraying air pressure 100 kPa and 500 kPa, pan air temperature 35°C and 55°C, and coating solution flow rate 2.2 g/min and 7.8 g/min.

In spite of development done in the past to improve the stability of drug formulation comprising aspirin by controlling the process parameters, there remains a need to develop more optimized and accurate process of preparation which provides the stable aspirin tablet formulation.

In one general aspect, there is provided a stable aspirin tablet comprising aspirin and one or more pharmaceutically acceptable excipients, prepared under controlled conditions of humidity.

In another general aspect, there is provided a stable aspirin tablet comprising aspirin and one or more pharmaceutically acceptable excipients wherein the free salicylic acid generated in the tablet is not more than 3.0% when the tablet is kept under stability testing for 6 months at 40°C and 75% relative humidity, and wherein the tablet is prepared under the conditions where relative humidity is maintained below 30%.

In another general aspect, there is provided a stable aspirin tablet comprising aspirin and one or more pharmaceutically acceptable excipients, prepared by dry granulation or direct compression methods wherein the free salicylic acid generated in the tablet is not more than 3.0% when the tablet is kept for stability testing for 6 months at 40°C and 75% relative humidity, and wherein the tablet is prepared under the conditions where relative humidity is maintained below 30%.

In another general aspect, there is provided a stable tablet composition comprising aspirin microcrystalline cellulose, starch pregelatinized, methacrylic acid ethyl acrylate copolymer (1:1), and one or more pharmaceutically acceptable excipients, wherein the free salicylic acid generated in the tablet is not more than 3.0% when the tablet is kept for stability testing for 6 months at 40°C and 75% relative humidity, and wherein the tablet is prepared under the conditions where relative humidity is maintained below 30%.

In another general aspect, there is provided a method of improving the stability of aspirin tablet wherein the free salicylic acid generated in the tablet is not more than 3.0% when the tablet is kept for stability testing for 6 months at 40°C and 75% relative humidity, and wherein the method comprises preparing tablet under the conditions where relative humidity is maintained below 30%.

The process of preparation of the present invention helps improve the stability of aspirin tablets. Tablets prepared under controlled humidity condition, have significantly improved stability of the final formulation when tested under stability conditions described herein. Thus, the stable aspirin tablets of the present invention are prepared under by controlled process parameters without the need to use any additive to control the hydrolytic degradation of aspirin. This technique for improving stability is simpler and less tedious as compared to that of those used in the art, and is more effective in controlling hydrolysis of aspirin.

The term "stable aspirin tablet" as used herein refers to the aspirin tablet wherein Free Salicylic Acid generated is not more than 3.0% when kept for stability testing (40°C, 75 % RH) for 6 months.

The term "aspirin" as used herein includes acetylsalicylic acid (aspirin) its pharmaceutically acceptable salts such as aspirin sodium, aspirin potassium, and the like. The amount of aspirin present in tablet may vary from about 10 mg to about 1000 mg.

The term "pharmaceutically acceptable excipient" as used herein includes all physiologically inert additives used in the pharmaceutical art of formulation. Examples include diluents, binders, disintegrants, lubricants, glidants, colorants, plasticizer, opacifiers, film-forming polymers, and the like.

Examples of diluents include microcrystalline cellulose, starch pregelatinized, corn starch, starch, cellulose powdered, dextrates, dextrins, dextrose excipients fructose, kaolin, lactitol, lactose, mannitol, sorbitol, sucrose, sugar compressible, sugar confectioners, and the like.

Microcrystalline cellulose is a purified, partially depolymerised cellulose, prepared by treating alpha-cellulose, obtained as a pulp from fibrous plant material, with mineral acids; used as a tablet diluent. Microcrystalline Cellulose has unique compressibility and carrying capacity. It exhibits excellent properties as an excipient for solid dosage forms. It compacts well under minimum compression pressures, has high binding capability, and creates tablets that are extremely hard, stable, yet disintegrate rapidly. Other advantages include low friability, inherent lubricity, and the highest dilution potential of all binders. Microcrystalline cellulose is available in various grades e.g. Avicel PH 101, 102, 301, 302 etc. Avicel PH-101 is the logical choice for intragranular incorporation as it ensures faster and more trouble-free processing. Avicel PH promotes rapid, even wetting; speeds drying; reduces screen blockage; minimized case hardening; controls dye migration; and promotes disintegration. On the other hand high density Avicel PH-301 or PH-302 as well as Avicel PH-102 may be used as additions to the running mix to improve flow and compatibility during compression. Microcrystalline cellulose may be incorporated in range of about 5% to about 60% of total weight of solid dosage form.

Corn starch is a cereal starch that is low in ash and protein content. A versatile additive, it suits the needs of several industries. Corn starch controls the aesthetics, moisture, texture, consistency and shelf-life of food products. It improves processing and finished food quality and ensures a consistent final product. Corn starch is most suited as a vehicle for tablet compression in the pharmaceutical industry. Corn starch is available under many brand names e.g. STA-RX® NF. STA-RX® NF is a corn based starch designed specifically for the pharmaceutical industry for tabletting. STA-RX® NF is available in many grades. It is used as a carrier for pharmaceutical solid dosage forms and may be incorporated in range of about 2% to about 30% of total weight of solid dosage form.

Examples of binders include pregelatinized starch, methyl cellulose, hydropropylcellulose, hydroxypropyl methylcellulose, carboxymethylcellulose, polyvinylpyrrolidone, gelatin, gum Arabic, ethylcellulose, polyvinyl alcohol, pullulan, agar, tragacanth, sodium alginate, and the like.

Examples of disintegrant include sodium starch glycolate, croscarmellose sodium, crospovidone, low substituted hydroxypropyl cellulose, and the like.

Examples of lubricants and glidants include talc, silica colloidal anhydrous, stearic acid, magnesium stearate, calcium stearate, hydrogenated castor oil, sucrose esters of fatty acid, microcrystalline wax, yellow beeswax, white beeswax, and the like.

Enteric coating layer over the aspirin core tablet may comprise enteric polymer with or without other coating additives. Examples of enteric polymers include cellulose acetate phthalate, hydroxypropyl methylcellulose acetate phthalate, polyvinyl acetate phthalate, hydroxy propyl phthalate, hydroxypropyl methylcellulose phthalate (HPMC phthalate), hydroxypropyl methylcellulose acetate succinate, cellulose acetate maleate, cellulose acetate scuccinate, methacrylic acid, ethacrylic acid and other acrylic acid esters, and copolymers thereof such as Eudragit® L 100-55 (methacrylic acid - ethyl acrylate copolymer (1:1) Type A" Ph. Eur.), Eudragit® L30 D-55, Eudragit® L 100, Eudragit® S 100; and mixtures thereof. In particular, Eudragit® L 30 D-55 may be used as the enteric polymer. Enteric coating is carried out till a weight build up of about 5% to about 15 %, is achieved over the core tablet.

Coating additives may be selected from the group comprising of plasticizers, coloring agents, lubricants/glidants, opacifiers, anti-foaming agent and the like.

Specific examples of plasticizers include acetylated triacetin, triethylcitrate, tributylcitrate, glyceroltributyrate, monoglyceride, rape oil, olive oil, sesame oil, acetyltributylcitrate, acetyltriethylcitrate, glycerin sorbitol, diethyloxalate, diethyl phthalate, diethylmalate, diethylfumarate, dibutylsuccinate, diethylmalonate, dioctylphthalate, dibutylsebacate, and the like.

Examples of colorants include any FDA approved colors for oral use.

Examples of opacifiers may include titanium dioxide, and the like.

Examples of anti-foaming agents include insoluble oils, dimethyl polysiloxanes (Simethicone emulsion) and other silicones, certain alcohols, stearates and glycols.

The stable tablet composition of aspirin is prepared by dry granulation/direct compression and further the tablets were coated. The whole process was performed under the conditions where RH is maintained below 30 %.

In one of the embodiments, a stable aspirin tablet may be prepared under the conditions where RH is maintained below 30 %, by the process comprising steps of:

  1. Sifting aspirin, and microcrystalline cellulose/corn starch,

  2. Blending the material of step 1,

  3. Sifting the pregelatinized starch and Silica colloidal anhydrous and adding to material of step 2,

  4. Sifting the stearic acid and adding to material of step 3 and blending,

  5. Compressing the blend of step 4,

  6. Preparing the enteric coating dispersion by adding and mixing talc, methacrylic acid-ethyl acrylate copolymer (1: 1), triethylcitrate and simethicone emulsion in water,

  7. Spraying the dispersion onto the tablet.

In another embodiment a stable aspirin tablet may be prepared under the conditions where RH is maintained below 30 %, by the process comprising steps of:

1. Sifting aspirin, and microcrystalline cellulose/corn starch,

2. Blending the material of step 1,

3. Sifting the pregelatinized starch and Silica colloidal anhydrous and adding to material of step 2,

4. Sifting the stearic acid and adding to material of step 3 and blending,

5. Compacting the material of step-5 in horizontal feed roller compactor,

6. Milling the compacts in oscillating granulator to achieve desired sized granules,

7. Compressing the granules of step 6,

8. Preparing the enteric coating dispersion by adding and mixing talc, methacrylic acid-ethyl acrylate copolymer (1: 1), triethylcitrate and simethicone emulsion in water,

9. Spraying the dispersion onto the tablet.

The invention is further illustrated by the following examples, which are not meant to limit the scope of the invention any way.

## Examples

**Table 1: Tablet composition**

| Ingredients | Amount (mg) | |
|---|---|---|
| | **Example I** | **Example II** |
| Aspirin | 75.00 | 75.00 |
| Microcrystalline Cellulose | 24.20 | 00.00 |
| Corn starch | 00.00 | 24.20 |
| Starch Pregelatinized | 7.70 | 7.70 |
| Silica Colloidal Anhydrous | 2.20 | 2.20 |
| Stearic Acid | 0.90 | 0.90 |
| Talc | 3.33 | 3.33 |
| Methacrylic Acid-Ethyl Acrylate Copolymer (1:1) | 10.50 (As dry substance) | 10.50 (As dry substance) |
| Triethyl Citrate | 1.05 | 1.05 |
| Simethicone Emulsion | 0.12 (As dry substance) | 0.12 (As dry substance) |
| Purified Water | q.s | q.s |

**Procedure**:

The whole process of preparation was performed under the conditions where RH is maintained below 30 %, by following steps:

1. Aspirin, microcrystalline cellulose/corn starch were sifted through appropriate sieve;

2. The above ingredients were mixed in a blender;

3. Pregelatinized starch silica colloidal anhydrous were sifted through appropriate sieve and added to material of step 2 and mixed in a blender;

4. Stearic acid was sifted through appropriate sieve;

5. Material of step 4 was added to the material of step 3 and mixed in a blender;

6. The above blend was compressed using approved punches and dies;

7. Enteric coating dispersion was prepared by adding and mixing talc, methacrlic acid-ethyl acrylate copolymer (1: 1), triethyl citrate, and simethicone emulsion in water; and

8. The dispersion was sprayed onto the tablets in a coating pan.

Aspirin tablets as per the composition of example I and II were also prepared under the normal relative humidity conditions (RH > 45 %) for comparative stability studies (Comparative Example I and Comparative Example II respectively).

**Stability Testing**

The tablets prepared as per the examples I and II and comparative examples I and II were packed in Aclar Blister packs and Cold form blisters and kept for stability testing under conditions given as below:

Temperature: 40°C

Relative Humidity: 75 %

Duration: 6 months

Products were tested for amount of salicylic acid generated in products

➢ At initial stage,

➢ At completion of 1 month,

➢ At completion of 3 months, and

➢ At completion of 6 months.

The results of the study are represented in Table 2.

**Tablet 2: Stability testing results**

| Example | Salicylic Acid (%) (initial stage) | Salicylic acid (%) 40°C 75 % RH (1 month) | | Salicylic acid (%) 40°C 75 % RH (3 month) | | Salicylic acid (%) 40°C 75 % RH (6 month) | |
|---|---|---|---|---|---|---|---|
| | | Cold form blister | Aclar Blister | Cold form blister | Aclar Blister | Cold form blister | Aclar Blister |
| Example I | 0.062 | 0.351 | 0.292 | 0.744 | 0.695 | 1.672 | 1.173 |
| Example II | 0.075 | 0.212 | 0.208 | 0.548 | 0.394 | 0.978 | 0.739 |
| Comparative Example I | 0.112 | - | - | - | - | 4.8 | - |
| Comparative Example II | 0.091 | - | - | - | - | 3.4 | - |

The tablets of comparative example 1 and 2 packed in Aclar Blister packs softened after 6 months during stability testing and needle shaped crystal growth was seen on the tablet surface, however there was no change in description of tablets in cold form blister.

The stability test data shows that aspirin degradation is much lower in tablets prepared under controlled dehumidified conditions in both packs.

While there have been shown and described what are the preferred embodiments of the invention, one skilled in the art will appreciate that various modifications in the invention may be made without departing from the scope of the invention as it is defined above.

**Claims**

1. A stable aspirin tablet comprising aspirin and one or more pharmaceutically acceptable excipients wherein the free salicylic acid generated in the tablet is not more than 3.0% when the tablet is kept for stability testing for 6 months at 40°C and 75% relative humidity, and wherein the relative humidity condition under which the tablet is prepared is maintained below 30%.

2. The stable aspirin tablet as claimed in claim 1, wherein the tablet is prepared by dry granulation or direct compression methods.

3. The stable aspirin tablet as claimed in claim 1, wherein the tablet comprises pharmaceutically acceptable excipients selected from diluents, disintegrants, binders, coating agents, lubricants, glidants, and colorants.

4. The stable aspirin tablet as claimed in claim 3, wherein the tablet comprises microcrystalline cellulose, starch pregelatinized, methacrylic acid ethyl acrylate copolymer (1:1), and one or more pharmaceutically acceptable excipients.

5. A method of improving the stability of aspirin tablet wherein the free salicylic acid generated in the tablet is not more than 3.0% when the tablet is kept for stability testing for 6 months at 40°C and 75% relative humidity, and wherein the method comprises preparing tablet under the conditions where relative humidity is maintained below 30%.

6. A stable aspirin tablet comprising aspirin and one or more pharmaceutically acceptable excipients, as described and illustrated in the examples herein.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4975283 A
- US 4900559 A
- US 4716042 A
- EP 0768082 A

**Non-patent literature cited in the description**

- **RUOTSALAINEN M et al.** *Pharm Dev Technol.,* 2003, vol. 8 (4), 443-51